# EUROPEAN PATENT SPECIFICATION

(11) **EP 0 890 338 B1**
(45) Date of publication and mention of the grant of the patent: **14.11.2007**
(21) Application number: 97305047.9
(22) Date of filing: 09.07.1997
(51) Int. Cl.: A61B 5/0402, A63F 13/02, A63F 13/10

(54) **Video game apparatus**
Videospielgerät
Appareil de jeu vidéo

(43) Date of publication of application: 13.01.1999
(73) Proprietor: Federal Patent Corporation, Miami, Florida 33181-2597 (US)
(72) Inventor: Clayman, Henry Mark, Miami, Florida 33181-2597 (US)
(74) Representative: Laight, Martin Harvey

(56) References cited:
- EP-A- 0 674 927
- WO-A-91/09374
- WO-A-96/14627
- US-A- 5 362 069
- US-A- 5 524 637

## Description

The present invention is directed to a video game.

Recent developments in computer technology have generated an explosion of video games to be played on personal computers and dedicated computer game systems to be played on home television systems. An abundance of diverse video games have been developed in the past twenty years.

U.S. Patents 5,362,069 and Reissue 34,728 each discloses an exercise device comprising a video game wherein a signal representing the heart rate of the player affects the level of play of the video game.

U.S. Patent 4,278,095 to Lapeyre discloses an exercise monitoring system. Lapeyre '095 discloses an exercise device which simply numerically displays a person's heart rate on a TV monitor. Lapeyre '095 does not incorporate a video game.

U.S. Patent 3,765,009 discloses an apparatus for displaying a time varying waveform. This reference discloses an apparatus to display a generic time varying waveform, including a person's cardio-waveform. However, it does not involve a video game. German Offenlegungschrift 2,822,434 discloses an exercise device wherein an exerciser's cardio-waveform is superimposed over a video display of a video tape or television broadcast. German '434 does not disclose a video game.

In WO 96/14627 (Home Healthcare Interactive, Inc.) there is disclosed an electronic health monitoring system which includes a modified CD-ROM multimedia interactive television video game console including microprocessor, hardware and software. One or more physiological data monitors are coupled to provide a signal representative of a user's physiological parameter to the multimedia processor through an isolated interface circuit. A hand held program controller with directional buttons is operated by the user to control the various functions of the multimedia processor. A television is coupled to the multimedia processor to provide sound and a video display based upon output signals from the multimedia processor. The video game console is modified to execute a variety of health related functions. There is no disclosure of apparatus constructed and arranged for playing a video game.

In WO 91/09374 (Hall-Tipping) there is disclosed an exercise device, such as an exercise bicycle, which is connected to a speed sensor indicating activity level, e.g. speed of the bicycle. The activity level signal, along with a heart rate signal, are provided to a video game, such as a Pac-man type video game. The game monitors the heart rate of the exerciser. If the heart rate falls outside preset minimum or maximum limits a certain action occurs in the game, such as an increase in speed or skill level of the opposition. In the Pac-man type game for example, should the heart rate fall below the desired workout rate, the villain (the goblin) would move at a speed faster than the players' speed, putting the player at a disadvantage. The player would respond by increasing his level of physical activity, thereby increasing the heart rate until it exceeds the minimum aerobic level required, at which time the villain's speed would return to its normal level.

There is disclosed in WO 91/09374 (Hall-Tipping) apparatus for playing a video game to be played by at least one player, said apparatus comprising control means for allowing said at least one player to provide an input to the video game by physical interaction with the control means; processing means for processing signals to allow the game to be played, said processing means being constructed and arranged to generate a video game; display means for displaying characteristics of the video game during playing; and sensing means for sensing at least one parameter of a bodily function of said at least one player to produce a signal representative of said at least one parameter, the processing means being connected to receive signals from the control means and from the sensing means. The sensing means is constructed and arranged to sense the cardio-waveform of said at least one player and to provide a signal representative of said cardio-waveform.

In accordance with the present invention such apparatus for playing a video game is characterised in that said processing means is constructed and arranged to display both the video game and the cardio-waveform and to integrate said cardio-waveform into the characteristics and activities of the video game.

In some forms of the apparatus, said at least one player comprises two players each an opponent to the other, and each of said at least two player's cardio-waveform is sensed and displayed.

It is preferred that said means for sensing said cardio-waveform of said at least one player is incorporated into said control means such that said player's cardio-waveform is sensed from simple contact between said at least one player and said control means.

In some forms of the apparatus said processing means is arranged to process and alter said signal representative of said at least one player's cardio-waveform to display a waveform which varies from a pure representation of a player's cardio-waveform.

In other forms of the apparatus, said processing means is arranged to process said signal representative of said cardio-waveform to display an unaltered representation of said at least one player's cardio-waveform. It may be arranged that said displayed unaltered cardio-waveform is superimposed over a predetermined cardio-waveform. In some forms of the apparatus it may be arranged that said processing means further comprises a transmission means for transmitting said signal representative of said at least one player's sensed cardio-waveform to a remote location so that said signal may be displayed on a remote electrocardiogram.

In one form of apparatus according to the present invention a conventional computer video game system is provided comprising a micro-processor based control system, a joystick or other manual control system and a video display system. A cardio-waveform sensor is also provided to sense a player's electrocardio waveform. This sensor is attached to a player's body, either independently or in conjunction with the game controls, and produces an electrical signal indicative of the player's cardio-waveform. This signal is transmitted to the input interface to enable processing by the micro-processor. The micro-processor uses the signal from the cardio sensor and joystick to generate and transmit signals to a video controller.

Embodiments of the invention will now be described by way of example with reference to the accompanying drawings in which:-
Figure 1 is a block diagram of a video game device embodying the invention with cardio-waveform and controller input devices;
Figure 2 is a representation of the video display illustrating the display of the time-varying cardio-waveform;
Figure 3 is a representation of the video display illustrating the display of the time varying cardio-waveform and a video game of the first embodiment;
Figure 4 is a representation of the video display illustrating the display of the time-varying cardio-waveform of increased frequency;
Figure 5 is a block diagram of a two player video game device according to an alternative embodiment of the claimed invention;
Figures 6a and 6b show a representation of a two player video display illustrating an alternative embodiment of the claimed invention; and

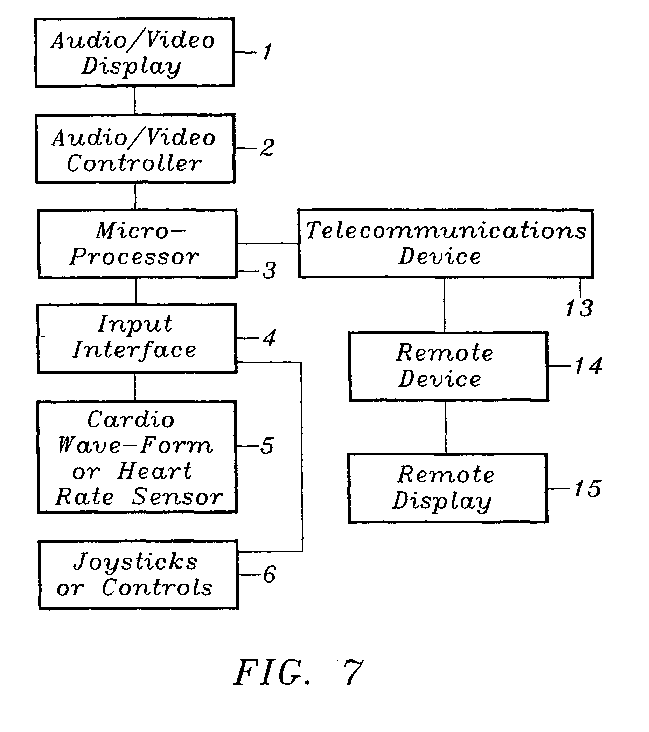

Figure 7 is a block diagram of an embodiment of a video game device according to the invention with a telecommunications device.

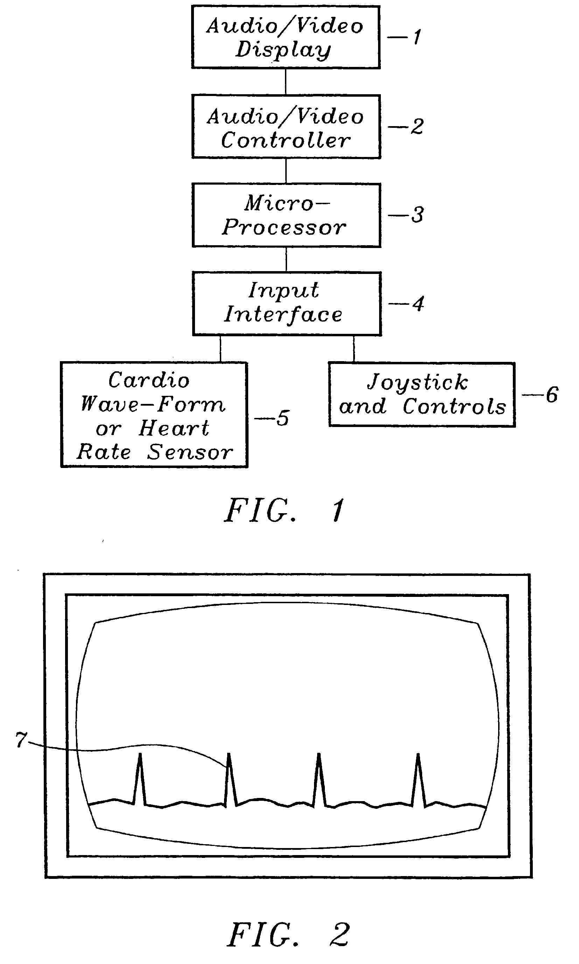

Figure 1 is a block diagram of a video display 1, audio/video controller 2, a micro-processor 3, input interface 4, manual joystick control 6, and cardio-waveform sensor 5. Cardio-waveform sensor 5, is attached to the video game player in a suitable portion of the player's body such as the chest area or on the inside area of the player's wrist. The preferred method of attaching the cardio-waveform sensor to the player's body is to the player's wrist where the waveform sensor 5 is embedded in a wrist band which is simply wound around the player's wrist and secured by Velcro (trademark) or the like. In an alternative embodiment, the sensor 5, is integrally formed with the manual control device 6 such that the player's cardio-waveform is sensed by simple physical contact between the player's hands and the controls. This embodiment relieves the necessity of having a separate dedicated connection to the player's body.

The cardio-waveform sensor 5 can be of any type of the well known sensors which send signals to a conventional Electro-Cardiogram (ECG). The cardio-waveform sensor 5 sends a signal, representative of the video game player's cardio-waveform, to input interface 4.

The input interface 4 also receives a signal from a manual control device 6. The manual control device 6 can be a joystick or other conventional video game control device which generates a signal in response to a player's physical movements. The input interface then sends a composite signal to the microprocessor 3 indicative of both the player's cardio-waveform and physical manipulation of the control device 6.

The micro-processor processes the signal sent from the input interface and incorporates the cardio-waveform signal into the particular characteristics and activities of the video game being played. The microprocessor then generates an audio and a video signal which are sent to the audio/video controller 2. The audio/video controller 2 then sends a display signal to the video display which displays the video game. A waveform 7 is displayed which not only represents the players time varying cardio-waveform but represents various characteristics of the video game being played as well. The audio/video controller also sends an audio signal to a speaker to produce sound. If the video game is to be played on a home television, a combined audio/video signal is sent to the television as conventionally found in the art.

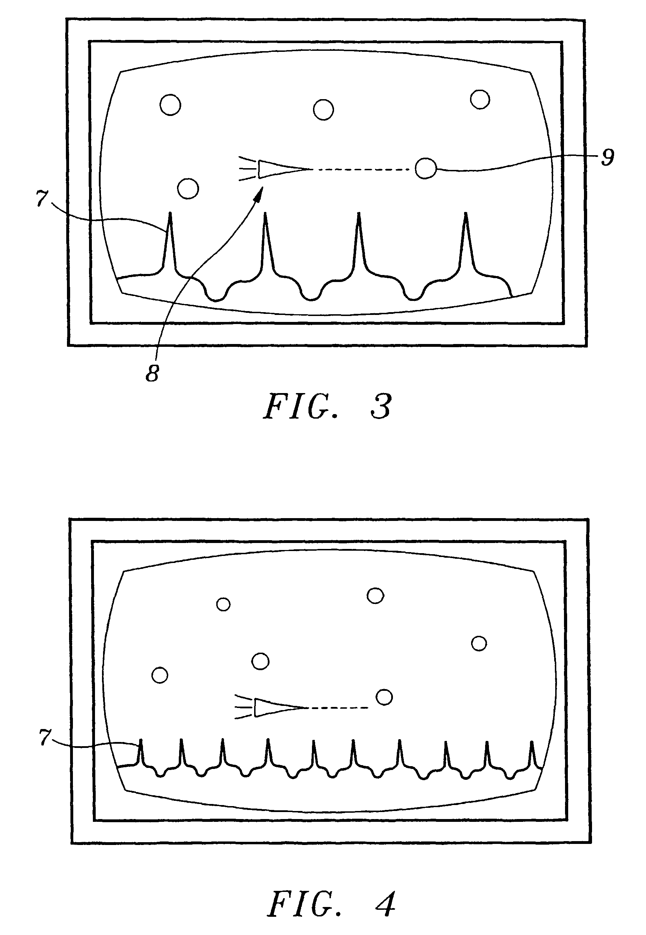

Figure 3 represents one embodiment of the claimed invention where waveform 7 represents a boundary which confines the activities of a roaming icon 8. Icon 8 can be of any type such as a space vehicle which must shoot obstacles 9 in its path or be destroyed. The movement of icon 8 is controlled by the manual control 6. In the event that the icon 8 collides with the waveform 7, the icon is also destroyed. Figure 4 represents the same video game as depicted in Figure 3, however the waveform 7 has increased frequency. In the event that the players heart rate increases, due to increased challenges of the video game for example, the frequency of the player's waveform will accordingly increase. This increased frequency will inherently provide less room for the roaming icon 8 to manoeuvre as the distance between the wave peaks is decreased. This will inherently pose an additional challenge to the player.

In an alternative embodiment of the claimed invention, the cardio-waveform can be altered to represent variations of the player's cardio-waveform. In one embodiment the frequency and amplitude of the waveform peaks can be increased or decreased to change the difficulty level of the game being played. The waveform displayed can vary from a pure representation of the player's cardio-waveform to a waveform which is merely a function of the player's cardio-waveform and thus altered by the particular characteristics a specific game to be played.

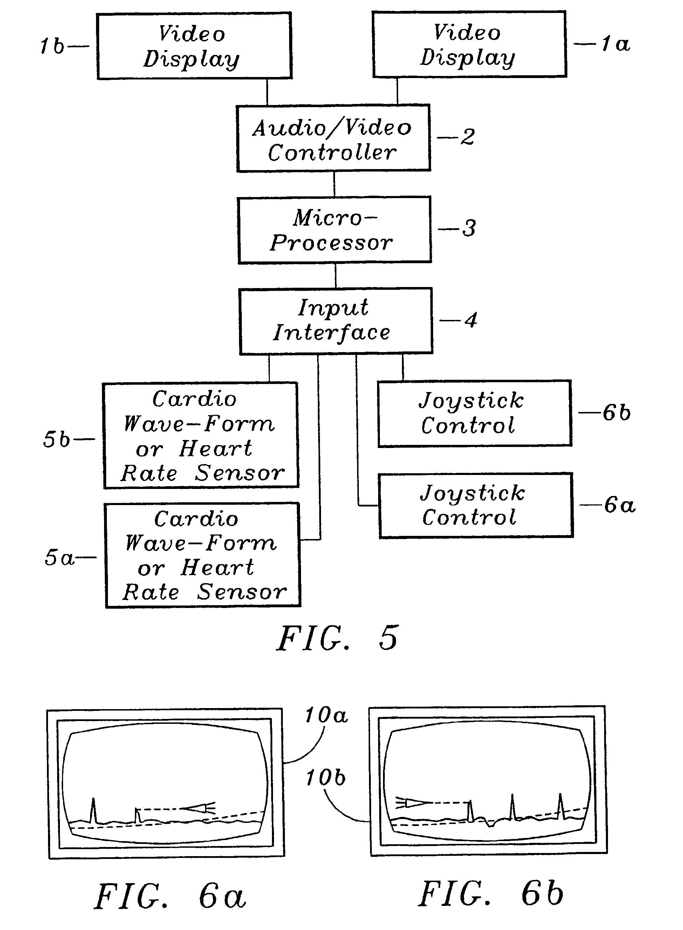

Figure 5, and Figures 6a, 6b represent an alternative embodiment of the claimed invention. Figure 5 represents a two player game where each player controls a joystick or other conventional control device 6a, 6b. Each player also is provided with a cardio-waveform sensor 5a, 5b. In the video game of this embodiment each player is provided with a video display 10a, 10b which displays their opponent's cardio-waveform and their roaming icon 8. The object of the game is to shoot the other player's waveform until it is flatlined or destroyed to below a threshold level. Each player manoeuvres their respective icon to shoot and destroy the opponent's waveform. In the event one player is successful in flatlining the opponent's waveform, the other player is killed. In the event that one player is flatlined the microprocessor can generate an audio signal indicative of a flat tone sound often associated with warning sounds found on heart rate monitors which sound an alarm when a person's heart stops.

The video game of the claimed invention may either be a self contained game system to be displayed on a conventional television unit or a computer game to be played on a personal computer. In the embodiment of the self contained system, it is preferred that the input interface 4, microprocessor 3 and video controller 2 are all housed in a single console. Signals are transmitted to the input interface 4 via the manual control 6 and the cardio-waveform sensor 5. The video controller then generates a signal capable of being displayed on a conventional television unit. In the personal computer embodiment, the signals from the manual control device 6, and the cardio-waveform sensor 5, are sent to the input interface when then sends corresponding signals to the microprocessor of the computer through the computer's serial port. The signals are then manipulated by appropriate software and the video game is displayed by the computers video controller.

The video game of the claimed invention, may also be provided with a diagnostic mode. When the diagnostic mode is selected the sensed cardio-waveform of the player is displayed in an unaltered form. This display affords the player an opportunity to view their cardio-waveforms and functions much like a conventional ECG. Information representative of normal readings may be stored in the read only memory (ROM) of the microprocessor to provide a comparative normal cardio-waveform from which to compare to the player's sensed cardio-waveform. In one embodiment the player's sensed waveform may be superimposed over the permanently stored normal waveform to facilitate diagnosis of irregularities in a player's cardio-waveform. In another embodiment, the video game can be provided with an output to send the sensed signal, representative of the player's cardio-waveform, to a telecommunications device 13 to then be transmitted to a remote device 14 for remote viewing on a remote display 15. In the event that a player self diagnoses an irregular cardio-waveform, the player may send the signal to a physician for a remote evaluation and accurate diagnosis. Such devices have currently been integrated into conventional ECGs but have not been integrated into a video game system

The cardio-waveform 7 can be integrated into an infinite number of types of video games limited only by the creativity of a video game programmer. In an infinite number of different embodiments, the sensed waveform can be manipulated to represent a landscape or other boundary from which a player's icon, an opponents icon or other icon must traverse, or depict a surface on which a player must try to manoeuvre an icon against. While the video game of this invention has been shown and described with reference to a particular embodiment, it will be understood to those possessing skill in the art that various changes to the form and detail may be made therein without departing from the scope of the invention.

Other features of the invention are set out in the accompanying claims which are to be read as being included in the description of this application.

## Claims

1. Apparatus for playing a video game to be played by at least one player, said apparatus comprising:
control means (6, 6a, 6b) for allowing said at least one player to provide an input to the video game by physical interaction with the control means;
processing means (3) for processing signals to allow the game to be played, said processing means being constructed and arranged to generate a video game;
display means (1, 1a, 1b) for displaying characteristics of the video game during playing; and
sensing means (5, 5a, 5b) for sensing at least one parameter of a bodily function of said at least one player to produce a signal representative of said at least one parameter, the processing means being connected to receive signals from the control means (6, 6a, 6b) and from the sensing means (5, 5a, 5b);
whereby said sensing means (5, 5a, 5b) is constructed and arranged to sense the cardio-waveform of said at least one player and to provide a signal representative of said cardio-waveform; **characterised in that** said processing means (3) is constructed and arranged to display both the video game and the cardio-waveform and to integrate said cardio-waveform into the characteristics and activities of the video game.

2. Apparatus as claimed in Claim 1 or 2, wherein said at least one player comprises two players each an opponent to the other, and wherein each of said at least two player's cardio-waveform is sensed and displayed.

3. Apparatus as claimed in Claim 1 or 2, wherein said means (5) for sensing said cardio-waveform of said at least one player is incorporated into said control means (6) such that said player's cardio-waveform is sensed from simple contact between said at least one player and said control means (6).

4. Apparatus as claimed in any preceding claim, wherein said processing means (3) is arranged to process and alter said signal representative of said at least one player's cardio-waveform to display a waveform which varies from a pure representation of a player's cardio-waveform.

5. Apparatus as claimed in any of Claims 1 to 3, wherein said processing means (3) is arranged to process said signal representative of said cardio-waveform to display an unaltered representation of said at least one player's cardio-waveform.

6. Apparatus as claimed in Claim 5, wherein said displayed unaltered cardio-waveform is superimposed over a predetermined cardio-waveform.

7. Apparatus as claimed in Claim 5 or 6, wherein said processing means further comprises a transmission means (13) for transmitting said signal representative of said at least one player's sensed cardio-waveform to a remote location so that said signal may be displayed on a remote electrocardiogram (15).

## Patentansprüche

1. Vorrichtung zum Spielen eines von mindestens einem Spieler zu spielenden Videospiels, die Folgendes umfasst:
Steuermittel (6, 6a, 6b), die es dem mindestens einen Spieler gestatten, durch physische Interaktion mit den Steuermitteln eine Eingabe an das Videospiel bereitzustellen;
Verarbeitungsmittel (3) zum Verarbeiten von Signalen, die das Spielen des Spiels gestatten, wobei die Verarbeitungsmittel so aufgebaut und angeordnet sind, dass sie ein Videospiel erzeugen;
Anzeigemittel (1, 1a, 1b) zum Anzeigen von Merkmalen des Videospiels während des Spiels; und
Erfassungsmittel (5, 5a, 5b) zum Erfassen mindestens eines Parameters einer körperlichen Funktion des mindestens einen Spielers, um ein Signal zu erzeugen, welches den mindestens einen Parameter darstellt, wobei die Verarbeitungsmittel angeschlossen sind, um Signale von den Steuermitteln (6, 6a, 6b) und von den Erfassungsmitteln (5, 5a, 5b) zu empfangen;
wobei die Erfassungsmittel (5, 5a, 5b) so aufgebaut und angeordnet sind, dass sie die Herzwellenform des mindestens einen Spielers erfassen und ein Signal, welches die Herzwellenform darstellt, bereitstellen; **dadurch gekennzeichnet, dass** die Verarbeitungsmittel (3) so aufgebaut und angeordnet sind, dass sie das Videospiel und die Herzwellenform anzeigen und die Herzwellenform in die Merkmale und Aktivitäten des Videospiels integrieren.

2. Vorrichtung nach Anspruch 1 oder 2, wobei der mindestens eine Spieler zwei Spieler, die jeweils Gegner sind, umfasst und jede der Wellenformen der mindestens zwei Spieler erfasst und angezeigt wird.

3. Vorrichtung nach Anspruch 1 oder 2, wobei die Mittel (5) zum Erfassen der Herzwellenform des mindestens einen Spielers in die Steuermittel (6) so eingebettet sind, dass die Herzwellenform des Spielers aus einem einfachen Kontakt zwischen dem mindestens einem Spieler und den Steuermitteln (6) erfasst wird.

4. Vorrichtung nach einem der vorangehenden Ansprüche, wobei die Verarbeitungsmittel (3) so angeordnet sind, dass sie das Signal, welches die Herzwellenform des mindestens einen Spielers darstellt, verarbeiten und verändern, um eine Wellenform anzuzeigen, die von einer reinen Darstellung der Herzwellenform eines Spielers variiert.

5. Vorrichtung nach einem der Ansprüche 1 bis 3, wobei die Verarbeitungsmittel (3) so angeordnet sind, dass sie das Signal, welches die Herzwellenform darstellt, verarbeiten, um eine unveränderte Darstellung der Herzwellenform des mindestens einen Spielers anzuzeigen.

6. Vorrichtung nach Anspruch 5, wobei die angezeigte, unveränderte Herzwellenform einer vorbestimmten Herzwellenform überlagert wird.

7. Vorrichtung nach Anspruch 5 oder 6, wobei die Verarbeitungsmittel ferner Sendemittel (13) zum Senden des Signals, welche die erfasste Herzwellenform des mindestens einen Spielers an einen entfernten Ort senden, so dass das Signal an einem entfernten Elektrokardiogramm (15) angezeigt werden kann.

## Revendications

1. Dispositif pour faire jouer un jeu vidéo destiné à être joué par au moins un joueur, ledit dispositif comprenant :
des moyens de commande (6, 6a, 6b) pour permettre audit au moins un joueur de fournir une entrée pour le jeu vidéo par interaction physique avec les moyens de commande ;
des moyens de traitement (3) pour traiter des signaux pour permettre au jeu d'être joué, lesdits moyens de traitement étant réalisés et agencés pour générer un jeu vidéo ;
des moyens d'affichage (1, 1a, 1b) pour visualiser des caractéristiques du jeu vidéo durant le jeu ; et
des moyens de détection (5, 5a, 5b) pour détecter au moins un paramètre d'une fonction corporelle dudit au moins un joueur pour produire un signal représentatif dudit au moins un paramètre, les moyens de traitement étant connectés pour recevoir des signaux provenant des moyens de commande (6, 6a, 6b) et des moyens de détection (5, 5a, 5b) ;
moyennant quoi lesdits moyens de détection (5, 5a, 5b) sont réalisés et agencés pour détecter la forme d'onde cardiaque dudit au moins un joueur et pour délivrer un signal représentatif de ladite forme d'onde cardiaque ; **caractérisé en ce que** lesdits moyens de traitement (3) sont réalisés et agencés pour visualiser à la fois le jeu vidéo et la forme d'onde cardiaque et pour intégrer ladite forme d'onde cardiaque dans les caractéristiques et les activités du jeu vidéo.

2. Dispositif selon la revendication 1 ou 2, dans lequel ledit au moins un joueur comprend deux joueurs chacun adversaire de l'autre, et dans lequel chaque forme d'onde cardiaque desdits au moins deux joueurs est détectée et visualisée.

3. Dispositif selon la revendication 1 ou 2, dans lequel lesdits moyens (5) pour détecter ladite forme d'onde cardiaque dudit au moins un joueur sont incorporés dans lesdits moyens de commande (6) de manière que ladite forme d'onde cardiaque du joueur soit détectée à partir du simple contact entre ledit au moins un joueur et lesdits moyens de commande (6).

4. Dispositif selon l'une quelconque des revendications précédentes, dans lequel lesdits moyens de traitement (3) sont agencés pour traiter et modifier ledit signal représentatif de ladite forme d'onde cardiaque de l'au moins un joueur pour visualiser une forme d'onde qui varie par rapport à une pure représentation d'une forme d'onde cardiaque du joueur.

5. Dispositif selon l'une quelconque des revendications 1 à 3, dans lequel lesdits moyens de traitement (3) sont agencés pour traiter ledit signal représentatif de ladite forme d'onde cardiaque pour visualiser une représentation non modifiée de ladite forme d'onde cardiaque de l'au moins un joueur.

6. Dispositif selon la revendication 5, dans lequel ladite forme d'onde cardiaque non modifiée visualisée est superposée à une forme d'onde cardiaque prédéterminée.

7. Dispositif selon la revendication 5 ou 6, dans lequel lesdits moyens de traitement comprennent en outre un moyen de transmission (13) pour transmettre ledit signal représentatif de ladite forme d'onde cardiaque détectée de l'au moins un joueur à un emplacement à distance de manière que ledit signal puisse être visualisé sur un électrocardiogramme à distance (15).
